# EUROPÄISCHE PATENTSCHRIFT

(11) **EP 2 500 057 B1**
(45) Veröffentlichungstag und Bekanntmachung des Hinweises auf die Patenterteilung: **29.10.2014**
(21) Anmeldenummer: 12001723.1
(22) Anmeldetag: 14.03.2012
(51) Int. Cl.: A61M 37/00

(54) **Vorrichtung zum präzisen repetierenden Einstechen von flüssigen Stoffen in definierte einstellbare Tiefen der Haut und Verfahren zur Einbringung der Stoffe in kosmetisch hinreichende, jedoch medizinisch geringstinvasive Tiefen**
Device for precise repeat injection of liquid materials to defined configurable depths of the skin and method for bringing the materials to cosmetically adequate depths with minimum invasion
Dispositif de piqûre précise répétée de matières liquides à des profondeurs réglables définies de la peau et procédé d'application des matières à des profondeurs suffisantes d'un point de vue cosmétique mais peu invasives d'un point de vue médical

(43) Veröffentlichungstag der Anmeldung: 19.09.2012
(73) Patentinhaber: Long-Time-Liner Conture Make-up GmbH, 80331 München (DE)
(72) Erfinder: Blank, Anton, 70173 Stuttgart (DE)

(56) Entgegenhaltungen:
- EP-A1- 1 618 915
- CN-Y- 201 426 920
- DE-A1-102008 031 907

## Beschreibung

Vorrichtung zum präzisen repetierenden Einstechen von flüssigen Stoffen in definierte einstellbare Tiefen der Haut und Verfahren zur Einbringung der Stoffe in kosmetisch hinreichende, jedoch medizinisch geringstinvasive Tiefen.

### Stand der Technik

Geräte zum Einstechen von Stoffen in die Haut werden zum Beispiel zum Einbringen von Pigmenten für permanentes Make-up (Pigmentieren) oder einer Tätowierung sowie kosmetischer und medizinischer Stoffe genutzt und bestehen üblicherweise aus einer Antriebseinheit (Handgerät) zur Erzeugung einer repetierenden axialen Bewegung, die auf ein Stechmittel, vorzugsweise auf eine Nadel oder eine Lanzette wirkt, die in einem Stechmittelführungselement, vorzugsweise in einer Hülse oder Düse, geführt wird, welche vorzugsweise mit dem einzubringenden Wirkstoff benetzt oder befüllt ist, um die Haut bei jeder Stechbewegung punktuell anzustechen, damit der Wirkstoff in die Haut eindringen kann.

Die in DE 10 2008 031 907.4 vom gleichen Erfinder beschriebene neuartige Nullpunkteichung der Nadel, die Führung der Nadelhülse direkt auf der Haut, anstatt der Nadel selbst, die Messung der Stechamplitude, die Regelung der Stechamplitude anhand der gemessenen Stechamplitude, die mit einem einstellbaren Sollwert verglichen und ausgeregelt wird, die mögliche höhere Stechfrequenz mit der damit verbundenen kürzeren Behandlungszeit sowie einer kürzeren Schmerzbelastung der behandelten Person und ein neuartiger Resonanzantrieb ohne Vibrationen sowie ohne Lagerspiel, wurden in der Praxis realisiert und die realisierten Geräte werden von den Anwendern benutzt. Nachfolgende Erfindungen stellen Neuheiten und Weiterentwicklungen dar, die aus der praktischen Realisierung und Anwendung von DE 10 2008 031 907.4 erwachsen sind.

Das Stechmittel und die Stechmittelführungshülse stellen das mit der menschlichen Haut direkt in Berührung kommende Element dar, deshalb werden für eine hygienische Arbeit sterile Einmalprodukte verwendet, die nach der Behandlung entsorgt werden. Beim Stand der Technik wird durch eine elastische Feststoffmembran der Innenraum der Antriebseinheit (Antriebsbereich) vom Stechmittelbereich (Arbeitsbereich) gegen das Eindringen von gesundheitlich schädigenden Einflüssen separiert (Rücklaufsperre). Elastische Feststoffmembranen weisen erhebliche Nachteile für den Einsatz in einer Antriebseinheit auf, da ihre Abdichtung zu den beiden getrennten Bereichen, trotz der repetierenden Bewegung, selbst gegen die kleinsten gesundheitlich schädigenden Teilchen dicht sein müssen, die Membranen der Antriebseinheit einen erheblichen mechanischen Widerstand entgegen setzen, der durch höhere Antriebsleistung mit dem Nachteil einer größeren Erwärmung des Handgerätes (Antriebseinheit) ausgeglichen werden muss und dass sie eine im Verhältnis zum Stechmittel sehr große Dimension aufweisen müssen, um den erforderlichen Hub sowie eine möglichst große Elastizität zu erbringen. Diese in Bezug auf größtmögliche Hygiene bei der Anwendung zum Schutz des Endkunden und der Ergonomie für den Anwender einschränkenden Kriterien, limitieren darüber hinaus sowohl die Freiheit des Designs für ergonomisches Arbeiten als auch den Wirkungsgrad durch erhöhte Verlustleistung.

Nach dem Stand der Technik bestehen derzeit zwei verschiedene Verfahren der Bevorratung des Anwendungsstoffes der in die Haut eingebracht werden soll. Das erste Verfahren ist das Eintauchen der Stechmittelkombination in ein kleines mit dem einzubringenden Stoff gefülltes Töpfchen, beispielsweise als eine Art Fingerring getragen, um eine kleine Menge des Stoffes durch Benetzung und Kapillarwirkung aufzunehmen. Der Nachteil dieses Verfahrens besteht in der sehr geringen Menge an Anwendungsstoff, der pro Eintauchvorgang aufgenommen werden kann, da die Kapillarwirkung der Stechmittelführungshülse durch den Gegendruck der auf der anderen Seite durch den Rücklaufschutz geschlossenen Stechmittelführungshülse sehr begrenzt ist und das Benetzungsvolumen auf der kleinen Oberfläche der Stechmittelführungshülse ebenfalls sehr klein ist, so dass der Anwender sehr häufig neuen Anwendungsstoff aufnehmen muss. Das zweite Verfahren verwendet ein Depot für den Anwendungsstoff mit einem vergleichsweise großen Volumen, das in die Stechmittelführungshülse mündet, um ein Nachfüllen möglichst lange zu vermeiden. Der Nachteil dieses Verfahrens liegt in der nicht kontrollierbaren Mengenabgabe von Anwendungsstoff, so dass entweder zuwenig oder zuviel Anwendungsstoff aus der Stechmittelführungshülse auf die zu behandelnde Haut austritt, da die Austrittsgeschwindigkeit unter anderem von der Viskosität des Anwendungsstoffes, des Stechfrequenz und dem Stechmittelspiel in der Stechmittelführungshülse abhängt. Beide Verfahren genügen nicht ausreichend den Anforderungen nach hoher Qualität für einen gleichmäßigen Auftrag von Anwendungsstoff auf die Haut.

Die unterschiedlichen Ausführungen der Befestigung des Stechmittels an oder in der Antriebseinheit nach dem Stand der Technik reichen von Spannhülsen über Spannzangen, Kugelklemmungen, Rastungen, Bajonettverschlüssen und vielen weiteren Ausführungen, die den relativ geringen Stechfrequenzen genügen. Geräte mit Antriebseinheiten nach dem Resonanzprinzip erreichen jedoch Frequenzen, die eine sicherere Stechmittelklemmung benötigen.

Die Stechmittelkombinationen des Stands der Technik werden entweder einzeln oder gemeinsam in einer sterilisierten Kunststoff-Papierverpackung zur Verwendung bereit gestellt. Die am Markt befindlichen Stechmittel-module werden als ganze Einheit in einer ähnlichen sterilen Verpackung bereit gestellt. Stechmittelkombinationen, die als Einzelteile bereit gestellt werden, müssen ausgepackt und vom Anwender zusammengesetzt werden, was der Hygiene abträglich ist. Stechmittelkombinationen, die zusammengesetzt bereit gestellt werden, müssen ebenfalls ausgepackt und im ausgepackten Zustand in die Antriebseinheit eingefügt werden, was ebenfalls der Hygiene abträglich ist. Stechmittelmodule müssen ebenfalls ausgepackt werden und im ausgepackten Zustand in die Antriebseinheit eingefügt werden, gleichzeitig dient der verlängerte Schaft des Moduls als Griffstück für das gesamte Gerät, was nicht das Optimum an Hygiene darstellt.

Nach dem Stand der Technik bestehen während des Betriebszustands derzeit keinerlei mechanische oder elektrische Sicherheitseinrichtungen in Bezug auf Gerätestörungen der Antriebseinheit, das Lösen des Stechmittels oder der Stechmittelführungshülse aus der Antriebseinheit und der Begrenzung des Hubs der Stechmittels im Störungsfall oder bei einem Anwendungsfehler. Bei einem Defekt der Motorlagerung, des Getriebes, der Taumelscheibe oder des Hubmagnets nach dem Stand der Technik und beispielsweise einem Lösen des Stechmittels oder der Stechmittelkombination während der Anwendung, können dem Endkunden ernsthafte körperliche Schäden zugefügt werden, da beispielsweise beim Pigmentieren von Augenoberlidern sehr dicht am Auge gearbeitet wird.

Die derzeitige Praxis zur Ermittlung der Stechtiefe in der Haut wird nach der Überzeugung der Anwender und Hersteller grob näherungsweise durch Beobachtung von austretender Lymphflüssigkeit ermittelt, die ein Indikator für die Erreichung beziehungsweise Verletzung der Lederhaut ist, in die der Anwendungsstoff eingestochen werden muss. Dabei ist es von der Geschicklichkeit und Erfahrung des Anwenders abhängig, wie gleichmäßig er das freilaufende Stechmittel zu führen vermag. Es ist jedoch klar ersichtlich, dass dieses Verfahren jede Präzision vermissen lässt, denn die zu erreichende Lederhaut (Korium) liegt im Gesicht von Menschen bei 0,03 bis 0,20mm unter der Oberhaut (Epidermis). Eine derart geringe Tiefe von Hand einzustellen und auch noch konstant zu halten ist für Menschen schlichtweg unmöglich, denn die Hand zittert bereits mit einer vergleichbaren Amplitude der Dicke der Epidermis und die Antriebseinheiten, abgesehen von Resonanzantrieben, weisen Vibrationsamplituden von teilweise dem Mehrfachen der zu erreichenden und zu haltenden Einstechtiefe auf. Da die Lymphflüssigkeit der Haut zudem verzögert austritt, ist eine Einbringung von Anwendungsflüssigkeit in eine präzise Tiefe und ein gleichmäßiger Tiefenverlauf außer mit massenausgeglichenen Resonanzantrieben, Nullpunkteichung und Führung der Stechmittelführungshülse auf der Haut quasi unmöglich.

Die derzeit nach dem Stand der Technik verwendeten Geräte arbeiten bis auf wenige Ausnahmen mit separaten Versorgungs- und Steuereinheiten und einem Verbindungskabel, das beide Systeme elektrisch miteinander koppelt. Das Verbindungskabel stellt eine potenzielle Hygieneverschlechterung dar und ist zudem immer störend für den Anwender, da sich bei Anwendungen im Gesicht das Kabel immer im Wege befindet. Die wenigen mit Akkumulatoren betriebenen Antriebseinheiten, die auf ein Kabel verzichten können, sind so schwer und schwerpunktmäßig so ungünstig ausgewogen, dass ein Arbeiten sehr schnell zu Ermüdungserscheinungen der Hand führt. Zudem sind die Akkumulatoren fest in das Handgerät eingebaut und halten wegen der ungünstigen Effizienz der Antriebseinheiten nur ca. 30 Minuten. Der Anwender benötigt für eine fachgerechte Pigmentierung jedoch wesentlich länger, so dass er das Ladekabel in das Handgerät einstecken muss, um weiter arbeiten zu können. Damit sind sämtliche Vorteile von akkubetriebenen Geräten zunichte gemacht.

Der Stand der Technik in Bezug auf ein Komplettsystem ist beispielsweise im Dokument DE 10 2008 031 907.4 beschrieben, der Stand der Technik in Bezug auf ein Nadelsystem ist beispielsweise in EP 1 618 915 A1 beschrieben.

### Die Erfindung

Aufgabe der Erfindung ist es, eine verbesserte Präzision, Prozesssicherheit und Arbeitshygiene eines Gerätes zum Einstechen von Stoffen in definierte Tiefen der Haut zu ermöglichen und das Einbringen der Stoffe in kosmetisch hinreichende, jedoch medizinisch mit geringstinvasiver Verletzung, also in geringstmögliche Tiefen der Haut ermöglicht sowie den Anwendern bessere Nutzungseigenschaften bereit stellt und dem Endkunden eine kürzere, schmerzärmere und qualitativ höherwertige Behandlung zukommen lässt. Die genaue Steuerbarkeit der Eindringtiefe der Nadel in die Haut soll zudem eine beeinflussbare zeitliche Haltbarkeit der Stoffe in der Haut ermöglichen, so dass beispielsweise eine kürzere oder längere Haltbarkeit des permanenten Make-Up vorbestimmt werden kann, indem die Stoffe tiefer oder weniger tief in die Haut eingebracht werden.

Diese Aufgabe wird erfindungsgemäß durch eine Vorrichtung nach dem unabhängigen Anspruch 1 gelöst.

Das Stechmittel (7) und die Stechmittelführungshülse werden nicht wie beispielsweise in Dokument EP1618915A1 beschreiben als verbundene Einheit, also als Modul, ausgeführt, sondern als lose Einzelteile zusammengeführt. Die aus hygienischen Gründen vorteilhafte Trennung von Antriebseinheit und Stechmittelbereich (Rücklaufsperre) wird durch Einbringen einer geeigneten nicht verdampfenden hygienischen sterilisierbaren Flüssigkeit mit entsprechender Viskosität realisiert, die sich in dem der Antriebseinheit zugewandten Teil der Stechmittelkombination (10) befindet. Diese Flüssigkeitsdichtung löst sämtliche bisher bestehenden Probleme nach dem Stand der Technik. Sie schließt vollständig auch gegen kleinste Teilchen ab, bewirkt durch ihre Klebrigkeit eine Haftung der Fremdstoffe, setzt der Antriebseinheit keinen relevanten mechanischen Widerstand entgegen, vermeidet daher den Bedarf an höherer Antriebsleistung zum Ausgleich dieses Widerstands und der damit verbundenen höheren Erwärmung lässt sich nahezu beliebig klein ausführen, passt sich jeder durch das Design und die Ergonomie erforderlichen Form an und mindert den Wirkungsgrad des Antriebs nicht.

Die Stechmittelklemmung wird insbesondere für Antriebseinheiten mit Resonanzantrieb vorteilhafterweise in einem Arbeitsgang zusammen mit der Nullpunktjustierung des Stechmittels beim Einführen der Kombination aus Stechmittel und Stechmittelführungshülse in die Aufnahme der Antriebseinheit vorgenommen. Die Befestigung der Nadel erfolgt durch eine von außen durch den Anwender bedienbare Schraube (9), die derart ausgeführt ist, dass sie in gelöstem Zustand eine Fixierung des Resonanzarmes in dessen Nullstellung bewirkt, um den Nullpunkt zu eichen und beim Einschrauben der Schraube das Stechmittel durch Schraubklemmung am Resonanzarm mit im Vergleich zu Stechmittelklemmungen des Stands der Technik, die durch Herausziehen gelöst werden können, höheren Kraft fixiert. Das Stechmittel kann nach der Schraubklemmung nicht mehr, wie nach dem Stand der Technik, von Hand herausgezogen werden. Die Gleichzeitigkeit der Befestigung des Stechmittels am Resonanzarm sowie die für die Tiefeneinstellung erforderliche Nullpunkteichung des Stechmittels, stellt dem Anwender eine erleichterte Bedienung und für den Anwender als auch den Endkunden eine erhöhte Sicherheit in Bezug auf die Befestigung der Nadel bereit.

Bei einer vorteilhaften Ausgestaltung der Erfindung ist vorgesehen, dass die Stechmittelkombination, also das Stechmittel, die Stechmittelführungshülse und die Flüssigkeitsdichtung, zusammen in ein elastisches Behältnis, beispielsweise in einem kleinen elastischen Schlauch mit beidseitigen Verschlussstopfen sterilisiert zur Anwendung bereit gestellt wird. Der leichter entnehmbare, weil dünner ausgeführte Stopfen, welcher der Antriebseinheit zugewandt ist, wird durch Festhalten des gegenüber liegenden Stopfens abgezogen und die Stechmittelkombination in die Antriebseinheit eingefügt ohne dass irgendein Teil der Stechmittelkombination berührt werden muss. Beim Entfernen der Einweg-Stechmittelkombination wird der noch einseitig verschlossene Schlauch über die Stechmittelkombination gestülpt und diese wird entnommen, ohne dass irgendein Teil der Stechmittelkombination berührt werden muss. Diese geschlossene Hygienekette ist das Optimum an Hygiene und Sicherheit für den Anwender und den Endkunden.

Eine Fortbildung der Erfindung sieht vor, dass eine maximale mechanische Hubbegrenzung (5) der Antriebseinheit von 0,1-0,2mm über dem maximalen Nutzhub installiert wird, ein elektrischer Stechmitteldetektor (6) die Antriebseinheit sofort stoppt, sobald das Stechmittel keinen elektrischen Kontakt mehr zu einem zentral und größenmäßig begrenzt angeordneten Kontakt der Antriebseinheit mehr hat, was gleichzeitig bedeutet, dass die Antriebseinheit bei nicht korrekt eingesetztem Stechmittel überhaupt nicht in Betrieb genommen werden kann, und die Antriebseinheit so elastisch ausgebildet ist, dass sie im Betrieb bei unüblich ansteigendem Stechwiderstand ihren Hub proportional zum Stechwiderstand bis auf Null verringert. Diese Sicherheitseinrichtungen tragen erheblich zum Schutz des Endkunden bei und stellen ein neues Qualitätskriterium für sicheres Pigmentieren dar.

Bei einer zweckmäßigen Ausgestaltung der Erfindung kann vorgesehen sein, dass die Stechmittelführungshülse selbst als Mikrodepot ausgeführt ist, um eine größere Menge an Anwendungsflüssigkeit als im ersten Verfahren nach [0006] aufzunehmen und weniger als im zweiten Verfahren nach [0006], indem die Stechmittelführungshülse zur Beseitigung des in [0006] genannten Kapillargegendrucks mit einer kleinen Druckentlastungsöffnung (8) versehen wird. Durch die nun beidseitig offne Stechmittelführungshülse kann die Kapillarwirkung ungehemmt eintreten und die aufgenommene Menge an Anwendungsflüssigkeit kann mit dem Abstand der Druckentlastungsöffnung zum offenen Ende eingestellt werden. Mit diesem Verfahren kann die gespeicherte und ausgegebene Menge an Anwendungsflüssigkeit ohne großen Einfluss von Variablen wie Viskosität, Stechfrequenz oder Stechmittelspiel optimal auf eine qualitativ hochwertige Anwendung ausgelegt werden.

Bei einer Ausgestaltung der Erfindung kann vorgesehen werden, dass ein nichtinvasives elektronisches Hautdickenmessgerät zur Anwendung von Geräten zum Einstechen von Flüssigkeiten in die Haut zur Erhöhung der Anwendungspräzision sehr vorteilhaft ist.

Die in [0012] und [0015] des Dokuments DE 10 2008 031 907.4 aufgeführten Vorteile und Neuerungen werden in vorliegender Erfindung in nachfolgend aufgeführten Eigenschaften durch eine Neuerung ersetzt.

Der in [0012] im Dokument DE 10 2008 031 907.4 aufgeführte Resonanzaktor (1) wird dahingehend verbessert, dass er in unterschiedlichen Modi betrieben werden kann. Beim Stand der Technik wird der Resonanzaktor auf seiner Grundwelle betrieben, damit ist nur eine einzige Stechfrequenz möglich, die nicht für jede Hautregion oder Hautbeschaffenheit das Optimum an Schmerzfreiheit beziehungsweise an Farbeintrag bereit stellt. Durch den Betrieb des Resonanzaktors auf Harmonischen seiner Resonanzfrequenz (Grundwelle), können auf Basis der physikalischen Gesetze nach Fourier mit einem einzigen Resonanzaktor Vielfache der Resonanzfrequenz erzeugt werden. Bindet man den Massenausgleich zur dynamischen Auswuchtung von Vibrationen mit in das Resonanzsystem ein, so können aus einem einzigen Resonanzaktorsystem durch unterschiedliche Einstellungen der Resonanzfrequenzen beider Teilsysteme weitere Resonanzfrequenzen, die aus den Harmonischen des Resonanzaktors sowie aus den Harmonischen des Massenausgleichs bestehen, erzeugt werden. Damit ergeben sich nahezu beliebige Auslegungen des Resonanzsystems, die es gestatten, die erforderlichen unterschiedlichen Stechfrequenzen optimal an die kosmetischen und medizinischen Anforderungen anzupassen.

Die dem Anwender in [0015] im Dokument DE 10 2008 031 907.4 aufgeführte indikatorische Anzeige der Regeldifferenzen zwischen eingestellter und tatsächlicher Stichtiefe wird dahingehend verbessert, dass dem Anwender die tatsächliche Stechtiefe auf einem analogen oder digitalen Display (11) zusätzlich oder exklusiv angezeigt wird. Der Vorteil für den Anwender besteht darin, dass er außer beispielsweise der reinen Warnung, die Stechtiefe sei gerade nicht erreicht worden, zusätzlich und zeitlich parallel zu seiner Arbeit kontinuierliche Informationen über den tatsächlichen Tiefenverlauf erhält. Damit erkennt der Anwender momentan auftretende Unregelmäßigkeit sofort und auch ihrem Betrag nach und kann über die Tiefenanzeige korrigierend eingreifen. Die Qualität und Präzision bei der Herstellung von beispielsweise permanentem Make-Up wird dadurch weiter verbessert.

Bei einer vorteilhaften Ausgestaltung der Erfindung ist vorgesehen, dass die gesamte Regelungselektronik in der Antriebseinheit (2) eingebaut ist und das unhygienische und in der Anwendung störende Zuleitungskabel entfällt. Gleichwohl ist in diesem Fall die Stromversorgung als Akkumulator (3) ausgeführt, der vorteilhafterweise mit von der Antriebseinheit (4) lösbaren Wechselakkumulatoren gestaltet ist, um eine quasi kontinuierliche Anwendung zu gestatten. Der Einsatz neuer hochkapazitiver Akkumulatoren gestattet den Betrieb der Antriebseinheit (autarkes Handgerät) mit einer einzigen Akkuladung über Stunden, während zeitlich parallel der zweite Akku in einem Ladegerät aufgeladen wird. Mit diesem Verfahren kann daher quasi kontinuierlich beliebig lange gearbeitet werden.

### Bevorzugte Ausführungsbeispiele der Erfindung

Die Erfindung wird nachfolgend anhand eines Ausführungsbeispiels unter Bezugnahme auf die Figuren einer Zeichnung näher erläutert.

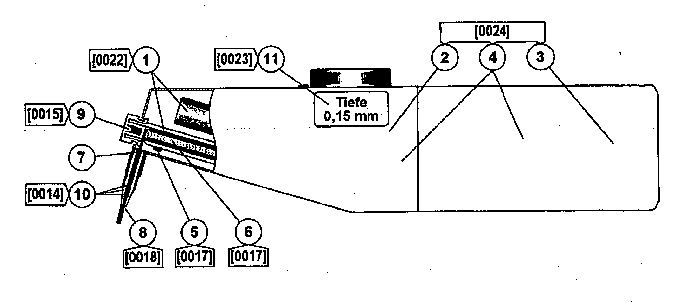

Fig. 1 Darstellung eines akkubetriebenen Gerätes zum präzisen repetierenden Einstechen von Stoffen in definierte einstellbare Tiefen der Haut sowie die efindungsgemäßen Neuerungen und Verbesserungen.

Fig. 1 zeigt eine Vorrichtung für präzises repetierendes Einstechen von flüssigen Stoffen in definierte einstellbare Tiefen der Haut. Die Beschriftung der einzelnen Neuerungen und Verbesserungen erfolgte zusätzlich analog der Nummerierung der Erfindung in vorliegendem Dokument, so dass die Zuordnung leichter fällt.

Die aus der Erfindung hervorgehenden Verbesserungen der Gerätepräzision, der Hygiene und der Qualität der Anwendung von Geräten zum Einstechen von Stoffen in die Haut sorgen für eine wesentlich verbesserte Gesamtqualität in Bezug auf die in [0004] bis [0011] des Stands der Technik angeführten Einflüsse.

Die in der vorstehenden Beschreibung, den Zeichnungen und den Ansprüchen offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. **Vorrichtung** zum präzisen repetierenden Einstechen von flüssigen Stoffen in definierte einstellbare Tiefen der Haut, mit
- einem Resonanzantrieb (1)
- einer Regelungseinrichtung und einem Wechselakkumulator (3) die in der Antriebseinheit (2) montiert sind,
- einer mechanischen Hubbegrenzung (5), einem vorzugsweise elektrischen Stechmitteldetektor und einem elastisch nachgebend ausgeführten Antrieb (1) ausgestattet ist,
- einem Stechmittel (7), dessen Nutz- und Antriebsbereich in der Stechmittelführungshülse durch eine Flüssigkeit hygienisch getrennt ist,
- einem Kapillardepot in der Stechmittelführungshülse mit einer Druckentlastungsöffnung (8),
- einer schraubbaren Nullpunktvorgebenden Klemmvorrichtung (9) für das Stechmittel, zur Befestigung des Stechmittels in der Antriebseinheit (2),
- einer vorzugsweise in einem Schlauch verschlossenen sterilen Stechmittetkombination (10), die vorbereitet ist, um in die Vorrichtung eingesetzt zu werden,
- einem analogen oder digitalen Display (11),
**dadurch gekennzeichnet, dass** der Resonanzantrieb (1) nicht nur auf seiner Grundwelle sondern auch auf seinen Harmonischen betrieben werden kann und der Massenausgleich des Antriebs zur dynamischen Auswuchtung von Vibrationen vorzugsweise eine andere Resonanzeinstellung aufweist, um mehrere Stechfrequenzen zu erzeugen, die optimal an die kosmetischen und medizinischen Anforderungen angepasst werden können.

2. Vorrichtung nach Anspruch 1, **dadurch gekennzeichnet, dass** der Betrieb der Antriebseinheit mit einem Wechselakkumulator (3) und eine in die Antriebseinheit (2) eingebaute Regelelektronik ein unhygienisches Kabel unnötig machen und durch die Verwendung neuer hochkapazitiver Wechselakkus ein quasi kontinuierliches, beliebig langes Arbeiten möglich ist.

3. Vorrichtung nach Anspruch 1, **dadurch gekennzeichnet, dass** die mechanische Hubbegrenzung (5) 0,1-0,2mm über der maximalen Einstechtiefe wirksam wird, der elektrische Stechmitteldetektor (6) die Antriebseinheit abschaltet, falls das Stechmittel den Kontakt der Klemmvorrichtung nicht mehr berührt oder dass die Antriebseinheit nicht anläuft, fall das Stechmittel nicht richtig eingesetzt ist und der elastisch ausgeführte Antrieb (1) bei einem unüblich ansteigenden Stechwiderstand seinen Hub proportional zum Stechwiderstand bis auf Null verringert.

4. Vorrichtung nach Anspruch 1 und 2, **dadurch gekennzeichnet, dass** die Stechmittelkombination (10) eine Flüssigkeit zur hygienischen Trennung des Stechmittelbereichs von der Antriebseinheit (2) gegen Teilchen jeglicher Größe bewirkt.

5. Vorrichtung nach den Ansprüchen 1 bis 3, **dadurch gekennzeichnet, dass** die Druckentlastungsöffnung (8) dafür sorgt, dass die Kapillarwirkung in der einseitig geschlossenen Stechmittelführungshülse wirken kann.

6. Vorrichtung nach Anspruch 1 bis 4, **dadurch gekennzeichnet, dass** die schraubbare Nullpunktvorgebende Stechmittel-Klemmvorrichtung (9) so gestaltet ist, dass das Stechmittel nach dem Klemmen auf den Nullpunkt der Schwingamplitude kalibriert ist und das Stechmittel (7) nach der Klemmung nicht mehr von Hand herausgezogen werden kann.

7. Vorrichtung nach den Ansprüchen 1 bis 5, **dadurch gekennzeichnet, dass** die sterile Stechmittelkombination (10) so gestaltet ist, dass das Einsetzen und das Entnehmen der Stechmittekombination berührungslos möglich sind.

8. Vorrichtung nach den Ansprüchen 1 bis 6, **dadurch gekennzeichnet, dass** das analoge oder digitale Display (11) die tatsächliche Stechtiefe kontinuierlich anzeigt.

## Claims

1. A device for the accurate repetitive insertion of liquids into the skin at defined adjustable depths, with
- a resonant drive (1)
- a control device and an interchangeable battery (3) that are mounted in the drive unit (2),
- a mechanical stroke limiter (5), a preferably electric puncturing-medium detector and an elastically yielding drive (1),
- a puncturing medium (7), the working and drive areas of which are hygienically separated in the puncturing-medium guide sleeve by means of a liquid,
- a capillary store in the puncturing-medium guide sleeve with a pressure-relief outlet (8),
- a screwable zero-point-indicating clamping device (9) for the puncturing medium, for attaching the puncturing medium to the drive unit (2),
- a sterile puncturing-medium combination (10), preferably sealed in a sterile tube, prepared so as to be inserted into the device,
- an analogue or digital display (11),
wherein the resonant drive (1) can be operated not only at its fundamental frequency but also at its harmonic frequencies, and wherein the mass balancing of the drive for the dynamic balancing of vibrations preferably has a different resonance setting in order to produce numerous puncturing frequencies, which can be adjusted for cosmetic and medical requirements.

2. A device according to claim 1, wherein the operation of the drive unit with an interchangeable battery (3) and a built-in electronic control circuit in the drive unit (2) make an unhygienic cord unnecessary, and wherein, through the use of new high-capacity batteries, quasi-continuous, indefinitely long periods of operation are possible

3. A device according to claim 1, wherein the mechanical stroke limiter (5) is effective 0.1-0.2mm beyond the maximum insertion depth, the electric puncturing-medium detector (6) switches off the drive unit if the puncturing medium is no longer in contact with the clamping device, or wherein the drive unit will not start if the puncturing medium is not properly employed and the elastic drive (1) loses its stroke rate proportional to an unusually increasing puncture resistance down to zero.

4. A device according to claims 1 and 2, wherein the puncturing-medium combination (10) causes a liquid hygienically to separate the puncturing-medium area from the drive unit (2) against particles of any size.

5. A device according to claims 1 to 3, wherein the pressure-relief outlet (8) ensures that the capillary effect is present in the closed-end puncturing-medium guide sleeve.

6. A device according to claims 1 to 4, **wherein** the screwable zero-point-indicating clamping device (9) is designed in such a way that, after clamping, the puncturing medium is calibrated to the zero point of the vibration amplitude and, after clamping, the puncturing medium (7) can no longer be extracted by hand.

7. A device according to claims 1 to 5, wherein the sterile puncturing-medium combination (10) is designed in such a way that insertion and removal of the puncturing-medium combination are possible without contact.

8. A device according to claims 1 to 6, wherein the analogue or digital display (11) continuously indicates the actual penetration depth.

## Revendications

1. Le dispositif pour l'insertion précise et répétitive de liquides à des profondeurs de l'épiderme réglables et prédéfinies, comprenant
- un système de propulsion à résonance (1)
- un dispositif de commande et une pile amovible (3) montés dans l'unité motrice (2)
- un limiteur de course mécanique (5), avec un détecteur de moyen de perçage électrique de préférence, et un propulseur élastique fourni (1)
- un moyen de perçage (7), dont la zone d'utilisation et l'unité motrice sont séparés de façon hygiénique par un liquide dans la gaine de guidage.
- un dépôt capillaire dans la gaine de guidage du moyen de perçage avec un pertuis de décompression (8),
- un dispositif de serrage boulonné paramétré sur zéro (9) pour le moyen de perçage (10) servant à le fixer dans l'unité motrice (2),
- une entité composé du dispositif de perçage, couleur et force motrice, de préférence scellée dans un tube stérile (10), prêt à l'insertion dans le dispositif,
- un affichage analogique ou numérique (11),
est **caractérisé par le fait que** le système de propulsion à résonance (1) peut être utilisé non seulement à sa fréquence fondamentale, mais également à ses harmoniques, et que la compensation de masse pour l'équilibrage dynamique des vibrations présente de préférence un autre paramètre a fin de produire différentes fréquences de perçage optimisées et capables de répondre aux exigences spécifiques de la cosmétique et du médical.

2. L'appareil, selon la revendication 1, se **caractérise par le fait que** l'unité motrice fonctionne avec une pile amovible (3) plus un circuit électronique de réglage intégré dans l'unité motrice (2) qui rend le cordon non hygiénique inutile. De plus l'utilisation de nouveaux accumulateurs de haute performance changeables permettent un travail continue d'une durée quasiment illimité.

3. L'appareil, selon la revendication 1, **se caractérise par le fait que** le limiteur de course mécanique (5) 0,1-0,2mm est efficace au-delà de la profondeur maximale d'insertion, le détecteur électrique du moyen de perçage (6) se désactive automatiquement si celle-ci n'est plus en contact avec l'outil de serrage ou bien si l'unité motrice ne démarre pas parce que le moyen de perçage n'a pas été inséré correctement et que la propulsion élastique (1) rencontre une résistance au perçage inhabituelle qui provoquerait la réduction de sa course proportionnellement à la résistance jusqu'à zéro.

4. L'appareil, selon les revendications 1 et 2, se **caractérise par** le fait la zone d'action du moyen de perçage et l'unité motrice (2) au sein de l'entité composé du dispositif de perçage, couleur et force motrice, sont séparés de manière hygiénique par un liquide les protégeant des particules de toute taille.

5. L'appareil, selon les revendications 1 à 3, se **caractérise par le fait que** le pertuis de décompression (8) permet de s'assurer que la capillarité peut se réaliser dans la gaine de guidage du moyen de perçage à extrémité fermée.

6. L'appareil, selon les revendications 1 à 4, se **caractérise par le fait que** le dispositif de serrage de l'aiguille paramètre sur zéro (9) est conçu de telle sorte que le moyen de perçage soit calibrée une fois la pince réglée sur le point d'amplitude de vibration zéro, et que le moyen de perçage (7) après serrage, ne peut plus être retirée à la main.

7. L'appareil, selon les revendications 1 à 5, sa **caractérise par le fait que** l'entité stérile composé du dispositif de perçage, couleur et force motrice (10) est conçue de telle sorte que son insertion et son retrait sont possibles sans contact.

8. L'appareil, selon les revendications 1 à 6, se **caractérise par le fait que** l'affichage analogique ou numérique (11) indique constamment la profondeur de pénétration effective
